# EUROPEAN PATENT APPLICATION

(11) **EP 1 715 051 A1**
(43) Date of publication of application: **25.10.2006**
(21) Application number: 05008356.7
(22) Date of filing: 18.04.2005
(51) Int. Cl.: C12N 15/85, A61K 48/00, C12N 5/06, C12N 15/63

(54) **Gene construct systems for isolating a pure population of cells of a single cell type**

(71) Applicant: Schwarz, Florian, 80801 München (DE); Schwarz, Emanuel, 80799 München (DE)
(72) Inventor: Schwarz, Florian, 80801 München (DE); Schwarz, Emanuel, 80799 München (DE)
(74) Representative: Grünecker, Kinkeldey, Stockmair & Schwanhäusser Anwaltssozietät

(57) **Abstract**

The present invention relates to gene construct systems and vectors comprising said gene construct systems representing efficient tools to yield a highly pure population of a single functional transplantable cell type from a mixture of cells (a starting population). The invention further relates to a method for isolating a pure population of cells of a single cell type using the vectors according to the invention. Moreover, the present invention relates to a pharmaceutical composition comprising the gene construct system or vector or isolated cells of the invention, as well as to the use of same for tissue engineering, cell therapy or transplantation.

## Description

The present invention relates to gene construct systems and vectors comprising said gene construct systems representing efficient tools to yield a highly pure population of a single functional transplantable cell type from a mixture of cells (a starting population). The invention further relates to a method for isolating a pure population of cells of a single cell type using the vectors according to the invention. Moreover, the present invention relates to a pharmaceutical composition comprising the gene construct system or vector or isolated cells of the invention, as well as to the use of same for tissue engineering, cell therapy or transplantation.

### BACKGROUND OF THE INVENTION

Today's most urgent problem in transplantation is the lack of suitable donor organs and tissues. As the population ages, demands for organs and tissue therapies will only increase. In this context, tissue engineering promises to revolutionize the therapeutic possibilities of the future. In vitro grown tissues might one day be transplanted into the human body to enhance, repair or replace the function of damaged organs or tissues, especially in those organs where cells are thought to be in a postmitotic state.

One of the remaining unsolved problems is the cellular source for such in vitro grown tissues. Possible sources of cells are cells derived from the same individual (autologous), from the same species (allogeneic), or from a different species (xenogeneic), primary or immortalized cell lines and differentiated stem cells. Generally, autologous cells are thought to be advantageous but they are frequently unavailable, and efforts to expand them have been quite unsuccessful.

Embryonic stem (ES) cells are originally derived from cells of the inner cell mass of pre-implantation blastocysts and represent pluripotent undifferentiated cells capable of proliferation, self-renewal and the generation of large numbers of differentiated progenies. They promise to be an ideal and unlimited cellular source for tissue engineering.

ES cells are capable of spontaneously differentiating into derivatives of all three primary germ layers in vitro, hence providing cells which can be isolated and used for tissue engineering. Murine and presumably also human ES cells have the potential to develop along all lineages of the embryo. When returned to the embryonic environment by transfer into a host blastocyst or aggregation with blastomere stage embryos, ES cells behave like normal embryonic cells; they can contribute to all tissues in resulting chimeras including gametes. Thus, any sort of cell of the body could theoretically be derived from ES cells.

One of the main obstacles associated with ES cells, however, is the lack of a tool for differentiating ES cells into one specific cell type in vitro. When brought into an environment allowing differentiation, ES cells spontaneously differentiate into many different cell types - but none of these subpopulations of cells contributes to more than 5% of the whole population, unless a specific differentiation protocol is used. However, pure populations of cells would be a prerequisite for all strategies based on tissue engineering or direct cell transplantation, as introduction of contaminating pluripotent ES cells could give rise to teratomas. Teratoma formation due to injection of undifferentiating ES cells is broadly accepted as one of the defining criteria to determine the undifferentiated state of ES cells.

Efforts to provide an appropriate environment that directs the stem cells into one specific cell lineage, e.g. by adding specific cytokines or growth factors to the medium used for cultivation or differentiation, are described. These efforts were partly successful but are far from being able to provide any desired cell type.

Another attempt to direct differentiation of ES cells is to constitutively overexpress early transcription factors of the desired cell-lineage and thereby force the cells to differentiate into these lineage. This approach has several disadvantages: the cells derived from this method - even if differentiated into cells of the desired lineage - will continuously express the early transcription factor on a very high level, whereas normal cells usually stop the expression of early transcription factors at a given timepoint during development. As a consequence, the cells produced by overexpression of early transcription factors certainly do not perfectly resemble the cells that are desired. Another drawback is that very early transcription factors have to be used to ensure that the cell adopts the transcription profile of the desired cell lineage in every aspect, and final evidence that there is no earlier transcription factor in the desired cell lineage can never be given. Therefore, efforts based on the overexpression of transcription factors are not suitable to provide a desired cell type from ES cells.

In contrast, a rather promising approach to obtain pure populations of a specific cell type is the purification of the desired cells from a mixture of differentiating ES cells. As a prerequisite, the desired subpopulation of cells has to be specifically defined on a molecular level, e.g. by specific cell surface markers. Fluorescence-activated cell sorting (FACS) may then be used to purify the cells based on the expression of such a specific surface marker that is recognized by a fluorescent antibody.

Yet, in many cases, such as for cardiomyocytes, an appropriate endogenous marker is not known. In these cases, sorting methods will have to rely on the introduction of a (exogenous) marker gene under the control of a lineage-specific promoter. In this connection, the labelling of ventricular-like cardiomyocytes with the enhanced fluorescent green protein (EGFP), the expression of which is controlled by the tissue-specific 2.1 kb myosin light chain 2v promoter, has been described (Müller M. 2000). However, FACS is extremely slow and typically capable of analysing no more than 3000 cells per second for sorting purities of greater than 95% with yields of 50-70%. To achieve cell numbers required for transplantations into humans, a purification period of more than 500 hours would be necessary. Therefore, FACS does not appear be suitable to identify a rare population of cells or to separate large numbers of cells due to the excessive amount of analysis and sorting time. The need for an expensive cell sorter and the fact, that FACS-sorting is thought to alter the expression profile of the cells sorted are further disadvantages (David et al., 2005).

Alternative approaches relying on the introduction of a drug-resistance gene for antibiotic selection rather than a gene encoding a fluorescent protein are critical because of the long incubation period with the hazard of resistance and possible harmful effects of the antibiotic on terminally differentiated cells themselves. Additionally, both FACS based on fluorescent markers and selection based on antibiotic resistance rely on the expression of non-human genes, resulting in the presence of exogenous proteins in the cells that may cause immunological problems or even be toxic in patients.

It is well established that in developing mice, cell lineages can be marked with site-specific-recombinase (SSR)-systems like Cre/loxP or Flp/FRP. Cre and Flp are able to recombine DNA at specific target sites termed loxP and FRT with high fidelity and without the need for cofactors in actively dividing and postmitotic cells as well as in almost all tissue types. LoxP and FRT are distinct at the nucleotide level but share an overall structure which includes two 13 bp palindromic sequences separated by an 8 bp asymmetric spacer-sequence that enables an orientation of the whole target site. The DNA between two directly repeated target sites is excised by Cre and Flp as a circular molecule. This excision is effectively irreversibly due to the loss of the circular reaction product. In mice, an enhanced version of Flp, called Flpe, readily mediates recombination between FRTs separated by as little as a few kilobases or as much as 150 kb. SSR-systems have already been described for the purpose of cell lineage tracing and conditional gene inactivation in mice.

The systems currently used for cell lineage tracing express Cre or Flp under the control of a tissue-specific promoter. Usually, those systems involve two mouse strains that are intercrossed: (1) a "recombinase mouse", expressing Cre or Flp in a gene-specific fashion and (2) an "indicator mouse", harbouring a transgene that indicates that a recombination event has occurred and provides a permanent record of this event by transforming it into a heritable lineage marker. The basic elements contained within the indicator transgene are (a) a reporter gene (e.g. lacZ or EGFP), functionally silenced by insertion of a STOP cassette (containing, for instance, a head-to-tail array of four similian virus 40 (SV40) polyA sequences coupled with translational stop codons in all reading frames) located between two target sites (that is loxP or FRT, referred to as floxed or flrted); and (b) a broadly active promoter capable of driving expression ideally in all cell types and at all stages of development, such that following a recombination event in any given cell, said cell and all progeny cells will be marked regardless of their subsequent fate. In recombinase/indicator double-transgenic animals, Cre or Flp activity tissue-specifically removes the floxed or flrted stop-cassette between the broadly active promoter and the marker gene and irreversibly activates the expression of the reporter gene (marker gene). As the excision event activates the reporter gene at the level of chromosomal DNA, it is stably inherited to progeny cells, thereby marking these cells and revealing their contribution to embryonic and adult tissues.

A recent example for tissue-specific cell-lineage marking was described by Laugwitz et al. (2005), wherein Cre is expressed under the control of the isl1+ promoter and excises a stop-cassette between the endogenous (broadly active) Rosa26 promoter and the lacz gene. As a consequence, only cardiac precursor cells and their derivatives express β-galactosidase detected by X-gal (5-bromo-4-chloro-3-indolyl-β-D-galactosidase) staining.

More recently, it has been confirmed by Schindehütte et al. (2005) that Cre/loxP is a very reliable tool for cell lineage marking. The authors demonstrated that Cre (expressed under control of a specific promoter) excises an interrupting cassette between a β-actin-promoter and a marker gene, such as the gene encoding green fluorescent protein (GFP), in a tissue-specific way. In this context it has also been suggested that a Cre/IoxP/GFP-system could be used for the purification of a specific cell lineage. However; this system has several major drawbacks: It relies on the permanent introduction of a construct comprising exogenous genes (Cre itself and the marker protein) into the genome and it relies on FACS as the tool for cell separation. Therefore, all protocols for cell lineage sorting based on a simple Cre/IoxP/exogenous marker (e.g. lacZ or GFP) system as described above will not overcome the already described disadvantages.

Regarding SSR-systems, recently a new SSR-system has been described by Sauer et al. (2004), consisting of the site specific recombinase Dre which shows high homology to Cre and the specific recombinase sites rox. A further SSR-system (Φ31/ attB, attP) was described by (Groth et al. 2000, Belteki et al. 2003).

It is well established that silencing of a gene of choice can be accomplished by several means of post transcriptional gene silencing (PTGS): For instance, an antisense-RNA sequence may be used which hybridises with the mRNA of the targeted gene resulting in double-stranded RNA that can not be translated anymore. Even if controlled by a strong promoter a gene can efficiently be silenced using this method. It is broadly assumed that the main effect of the silencing achieved by generating a simple RNA sequence complementary to the targeted mRNA is not accomplished by a hybridisation of the two complementary RNA fragments and a withdrawal of the targeted mRNA from the ribosome, but by a cleavage of the double-stranded RNA into short (-22bp) fragments by RNAses specific for double-stranded RNA which initiate the RNA induced silencing complex (RISC; Hannon, 2002).

Another even more potential way of silencing a specific gene is based on RNA interference (RNAi). The RNAi effect can be mediated by transfecting cultured cells with short interfering RNA (siRNA), which is short, double-stranded RNA of 21 to 23 base pairs, or, more recently demonstrated, with short hairpin RNA (shRNA). siRNAs are usually present as double-stranded duplexes with two-nucleotide 38 overhangs and 58-phosphate termini. Although not fully understood, it has been shown that the effect of siRNA in eucaryotic cells is mediated both on a transcriptional level by methylation of the targeted gene, resulting in a weakening of transcription, and on a posttranscriptional level by triggering the assembly of a nuclease complex, commonly referred to as RISC, targeting homologous RNA for degradation resulting in a rapid degeneration of the targeted mRNA (Hannon, 2002). The shRNA contains a short sequence homologous to a part of the target mRNA (sense sequence, about 22 nucleotides long), a "loop" region (a spacer of about 8 nucleotides) and a sequence complementary to the same part of the target-mRNA sequence (anti-sense sequence). The hairpin structure of the shRNA is cleaved into siRNA leading the RISC to the target-mRNA.

It has been shown that also in undifferentiated ES cells RNA interference is a very powerful tool for silencing genes (Kunath, 2003).

Although very different on the nucleotide level, antisense-RNA sequences and siRNA sequences are currently assumed to be two embodiments of the same principle in molecular biology.

### DESCRIPTION OF THE INVENTION

In view of the above, the problem underlying the present invention is to provide an efficient tool to yield a highly pure population of a single functional transplantable cell type.

This problem is solved by the provision, in one aspect, of a gene construct system comprising the following elements:
a) a recombinase gene expression cassette comprising
   i) a nucleic acid sequence encoding a recombinase; and
   ii) a lineage-specific or tissue-specific promoter operably linked to said nucleic acid encoding the recombinase;
b) a strong promoter;
c) a nucleic acid sequence encoding a detectable marker;
d) at least one stop cassette; and
e) at least two recombinase target sites;
wherein the at least one stop cassette is arranged between the strong promoter and the nucleic acid sequence encoding the detectable marker.

The term "DNA sequence" refers to a nucleic acid sequence encoding the primary sequence of a polypeptide or protein.

The term "promoter" as used in the present invention is meant to include any transcriptional control unit capable of initiating transcription of a nucleic acid sequence resulting in the production of RNA and includes regulatory elements such as enhancers and other regions binding transcriptional control factors.

The term "tissue-specific / lineage-specific promoter" is intended to comprise any promoter which is activated (i.e. is involved in the transcription of a gene) in a specific subpopulation of cells while it is not activated in the cells not belonging to this subpopulation, so that the promoter characterizes this subpopulation unambiguously.

The said promoter is called "operably linked" to a nucleic acid sequence encoding a polypeptide if the two components (promoter and coding nucleic acid sequence) work together to achieve expression of the polynucleotide. If "operably linked" to a promoter, the said nucleic acid sequence encoding a polypeptide is "under the control of the promoter".

A promoter is a "strong promoter", if the amount of RNA generated by transcription of the nucleic acid sequence which is under the control of the promoter is high. A promoter is "broadly active" when it is active in most tissues of a multicellular organism most of the time.

The term "detectable marker" refers to a polypeptide which can be used to permanently or transiently label a subpopulation of cells amongst a population of cells, in a way that allows to determine whether a certain cell belongs to the labelled subpopulation or not by biological, chemical, physical or other means.

The term "stop cassette" is used to refer to any intervening nucleic acid sequence that prevents operable linkage of a promoter with a nucleic acid sequence, thus inhibiting expression of said nucleic acid sequence.

The term "flanked by" means "located between" or "arranged between". All three terms are used interchangeably.

In one embodiment, the at least one stop cassette is located between the at least two recombinase target sites in the gene construct system of aspect one. Upon activation of the lineage-specific or tissue-specific promoter, the recombinase comprised in said gene construct system is expressed and removes the stop cassette located between the recombinase target sites, resulting in the expression of the nucleic acid sequence encoding the detectable marker under control of the strong promoter.

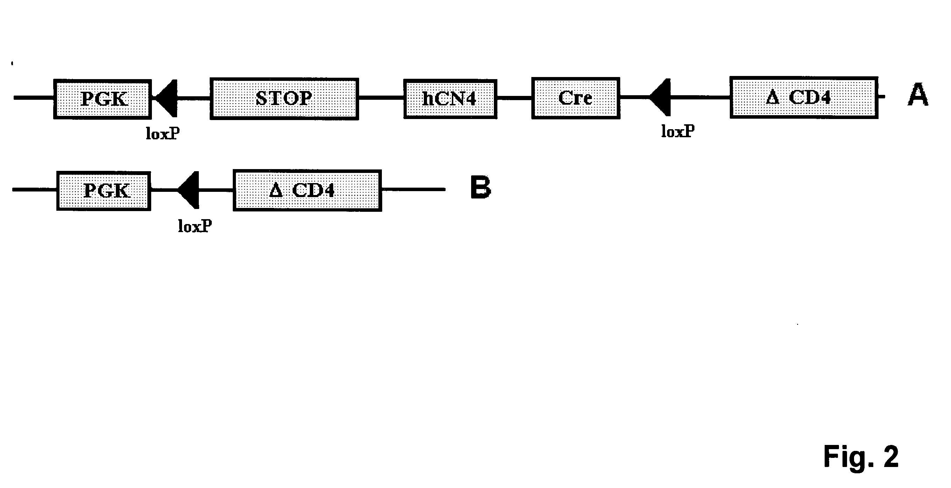

In another preferred embodiment, the recombinase gene expression cassette and the at least one stop cassette are located between the at least two recombinase target sites in the gene construct system of aspect one (an example of this embodiment is given in Fig. 2a). Upon activation of the lineage-specific or tissue-specific promoter, the recombinase comprised in said gene construct system is expressed and removes the recombinase gene expression cassette and the at least one stop cassette, resulting in the expression of the nucleic acid sequence encoding the detectable marker under control of the strong promoter (see Fig. 2b).

In a second aspect of the invention, a gene construct system comprising the following elements is provided:
a) a recombinase gene expression cassette comprising
   (i) a nucleic acid sequence encoding a recombinase; and
   (ii) a lineage-specific or tissue-specific promoter operably linked to said nucleic acid sequence encoding the recombinase
b) a strong promoter;
c) a nucleic acid sequence encoding a detectable marker;
d) at least one stop cassette; and
e) at least two recombinase target sites;
wherein one of the at least two recombinase target sites is arranged between the strong promoter and the nucleic acid sequence encoding the detectable marker.

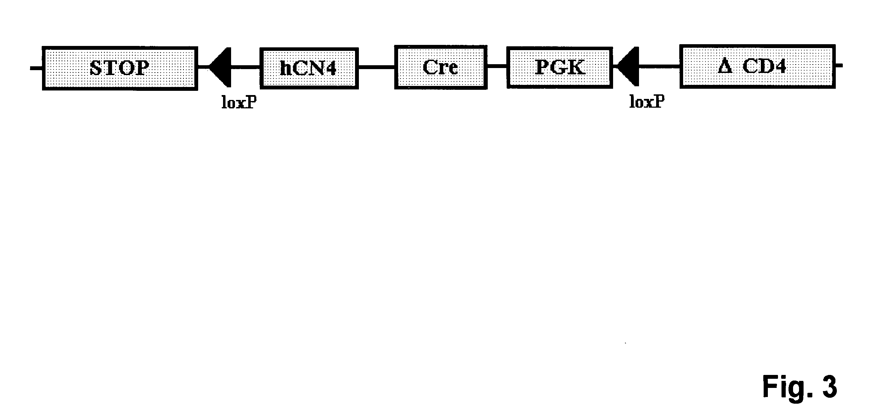

In a preferred embodiment of this second aspect, the recombinase gene expression cassette and the strong promoter are located between the at least two recombinase target sites (an example of this embodiment is given in Fig. 3). Upon on activation of the lineage-specific or tissue-specific promoter, the recombinase is expressed and removes the recombinase gene expression cassette and the strong promoter located between the recombinase target sites, resulting in a stop of expression of the nucleic acid sequence encoding the detectable marker.

In third aspect of the invention, a gene construct system comprising the following elements is provided:
a) a recombinase gene expression cassette comprising
   (i) a gene encoding a recombinase; and
   (ii) a lineage-specific or tissue-specific promoter operably linked to said gene encoding the recombinase;
b) at least one nucleic acid sequence encoding an antisense RNA or an siRNA or an shRNA directed against the marker mRNA
c) at least two strong promoters;
d) a marker gene encoding a detectable marker;
e) at least one stop cassette; and
f) at least two recombinase target sites;
wherein one of the at least two strong promoters is operably linked to the nucleic acid sequence encoding the marker, and one of the at least two recombinase target sites is arranged between the second of the at least two strong promoters and the nucleic acid sequence encoding the RNA sequence.

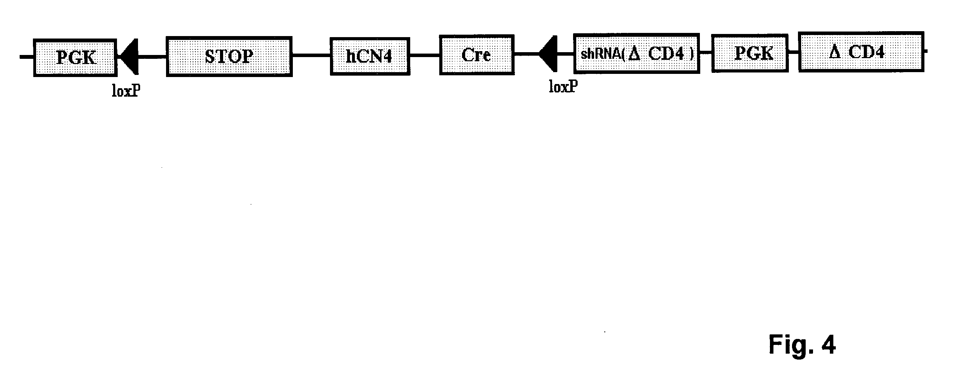

In a preferred embodiment of this aspect, the recombinase gene expression cassette and the at least one stop cassette are located between the at least two recombinase target sites (an example of this embodiment is given in Fig. 4).

The terms "antisense RNA directed against the marker mRNA" and "siRNA directed against the marker mRNA" and "shRNA directed against the marker mRNA" each refer to an RNA molecule capable of hybridizing to the mRNA produced by transcription of the nucleic acid sequence encoding the marker, leading to an efficient silencing of the targeted gene, on a transcriptional and/or a posftranscriptional level.

In a fourth aspect of the invention, a gene construct system comprising the following elements is provided:
a) a recombinase gene expression cassette comprising
   (i) a nucleic acid sequence encoding a first recombinase; and
   (ii) a lineage-specific or tissue-specific promoter operably linked to said nucleic acid sequence encoding the first recombinase;
b) at least two first recombinase target sites flanking the expression cassette of (a);
c) an inducilble promoter;
d) a nucleic acid sequence encoding a second recombinase;
e) at least two second recombinase target sites;
f) a strong promoter;
g) at least one stop cassette; and
h) a nucleic acid sequence encoding a detectable marker;
wherein at least one of the first recombinase target sites is arranged between the inducible promoter and the nucleic acid sequence encoding a second recombinase, and at least one of the second recombinase target sites is arranged between the strong promoter and the nucleic acid sequence encoding the detectable marker, and further
wherein the inducible promoter is specifically activated in the presence of a non-endogenous substance while the lineage-specific or tissue-specific promoter and the strong promoter are not activated.

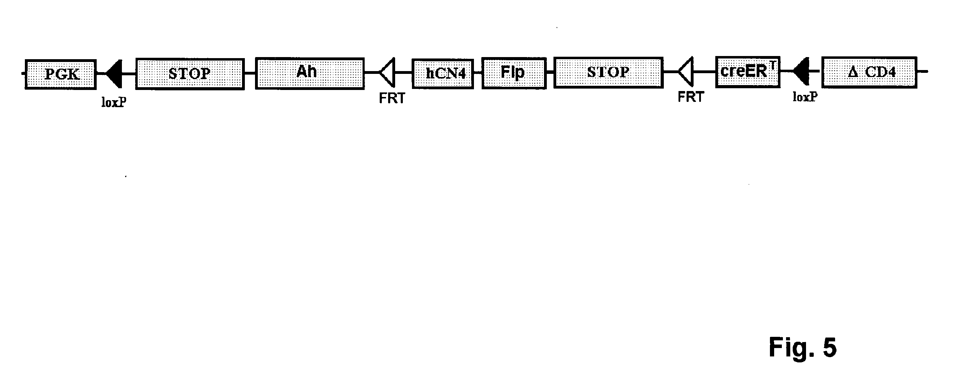

In a preferred embodiment of this aspect, the gene expression cassette of (a) flanked by the two first recombinase target sites recognized by the first recombinase is located between the inducible promoter and the nucleic acid sequence encoding the second recombinase, thus preventing expression from said inducible promoter; and the stop cassette, the inducible promoter, the nucleic acid sequence encoding the second recombinase, and the gene expression cassette of (a) flanked by the two first recombinase target sites are located between the strong promoter and the nucleic acid sequence encoding the marker, thus preventing transcription of the nucleic acid sequence encoding the marker (an example of this embodiment is given in Fig. 5).

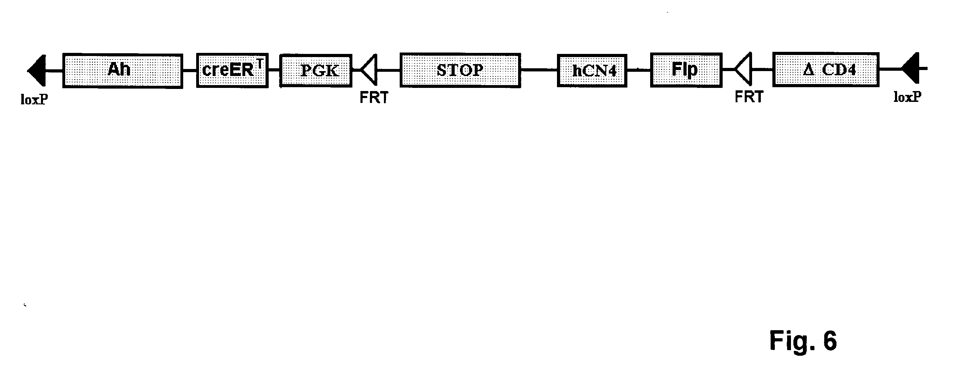

In a fifth aspect of the invention, a gene construct system comprising the following elements is provided:
a) a recombinase gene expression cassette comprising
   (i) a nucleic acid sequence encoding a first recombinase; and
   (ii) a lineage-specific or tissue-specific promoter operably linked to said nucleic acid sequence encoding the first recombinase;
b) at least one stop cassette;
c) at least two first recombinase target sites flanking the expression cassette of (a) and the stop cassette of (b);
d) a recombinase gene expression cassette comprising
   (i) a nucleic acid sequence encoding a second recombinase; and
   (ii) an inducible promoter operably linked to said nucleic acid sequence encoding the second recombinase an inducilble promoter;
e) at least two second recombinase target sites;
f) a strong promoter; and
g) a nucleic acid sequence encoding a detectable marker;
wherein at least one of the second recombinase target sites is arranged between the strong promoter and the nucleic acid sequence encoding the detectable marker, and further wherein the inducible promoter is specifically activated in the presence of a non-endogenous substance while the lineage-specific or tissue-specific promoter and the strong promoter are not activated (an example of this embodiment is given in Fig. 6).

Each of the gene construct systems according to the invention may consist of one or more constructs. That is, the elements of the specific gene construct system may be located on a single construct or on two (or more) different constructs. In a preferred embodiment, element (a) is included in the first of the at least two different constructs and elements (b)-(e) are included in the second of the at least two different constructs, and further the at least one stop cassette is arranged between the strong promoter and the nucleic acid sequence encoding the detectable marker and said stop cassette is flanked by the at least two recombinase target sites. In this preferred embodiment, the recombinase is preferably itself flanked by recombinase target sites.

Any site-specific-recombinase (SSR)-system may be used in the gene construct systems according to the invention. The use of the nucleic acid sequence encoding the recombinase Cre and the recombinase target sites loxP, and the nucleic acid sequence encoding the recombinase Flp and the recombinase target sites FRT are particularly preferred. Alternatively, the nucleic acid sequence encoding the Dre recombinase in combination with the recombinase target sites rox, or the nucleic acid sequence encoding the Φ31 recombinase in combination with the attB attP recombinase target sites may be used. Any variants of nucleic acid sequence encoding the recombinases may be used provided that said variants code for functional polypeptides showing recombinase activity; and any variants of recombinase target site sequences may be used, as long as they are recognized and subsequently cleaved by a functional recombinase.

The gene construct systems according to the invention may be used as a tool for positively or negatively marking any subpopulation of cells that activate a lineage-specific or tissue-specific promoter without the need of a permanent introduction of any non-human gene into the selected subpopulation of cells. The gene construct systems may be used to separate this subpopulation of cells from the cells of any starting population of cells (e.g. embryonic stem cells) that do not activate the lineage-specific or tissue-specific promoter.

If the subpopulation of cells to be selected is negatively marked by conditional excision of a strong promoter which controls expression of the marker or by conditional expression of antisense RNA or siRNA (see second and third aspect of the invention, Fig. 3 and 4), a surface marker is preferred that can be cleaved with the help of a specific enzyme, such as trypsin. Using a cleavable surface molecule as a marker, it is possible to remove the marker from the surface of every cell of the population following lineage-specific reassembly of the gene construct(s). After a while, all of the cells which do not belong to the subpopulation to be selected will express the marker, while the desired subpopulation will not express the marker and can thus be negatively selected. The use of intracellularly truncated CD4 as the marker and of trypsin as the cleaving enzyme are particularly preferred.

Preferably, the detectable marker used in the gene construct systems according to the invention is a surface-bound protein. An endogenous, "self" protein is particularly preferred. Generally any surface-bound protein that is not existent in the starting population of cells (e.g. in stem cells or early progenitor cells) can be used. That is, any surface-bound protein that is a "self" protein but that is not naturally displayed on any cell of the population from which the desired subpopulation is to be separated (or only in a very low amount) may serve as a marker which is unambiguously characterizing this subpopulation when introduced into the desired subpopulation.

If the existence of an immunogenic protein in the selected subpopulation is no impediment, a fluorescent protein such as EGFP may alternatively be used instead as a marker protein, thus enabling FACS-selection. Alternatively, an antibiotic marker such as a protein conferring resistance to neomycin or may be used.

According to the invention, the intracellularly truncated human CD4 molecule (ΔCD4) is particularly preferred as a surface-bound protein. As CD4 is an endogenous human protein, the expression of ΔCD4 on the surface of the cells makes an immunogenic potential of the marker protein in human unlikely. The surface-bound protein, such as ΔCD4, may be detected using antibodies tagged with magnetic beads and subsequently purified using magnetic cell sorting (MACS).

Further examples for proteins suitable in the constructs according to the invention are receptors such as the low affinity nerve growth factor receptor (LNGFR), MHC1 or any intracellularly truncated derivatives of these receptors (the truncation inhibiting signal transduction).

MACS is currently regarded to be the "Gold-Standard" for mild and time sparing cell purification. Using MACS, up to 10¹¹ cells can be analysed in about one hour, making it possible to separate large cell numbers and to identify even rare populations of cells. It is well established that with the help of MACS, cells transfected with a gene construct system can be positively selected, either depending on the expression of the gene construct system or independent from the expression of the construct thus selecting only for a successful transfection. For both purposes, e.g. ΔCD4 can be used as a marker. ΔCD4 can for instance be added to the gene of interest with the help of an internal ribosome entry site (IRES) so that a bicistronic mRNA is constructed whenever the gene of interest is transcribed and ΔCD4 is expressed in the same amount as the gene of interest, thus enabling positive selection for expression of the gene construct system . Alternatively, ΔCD4 can be placed under control of a broadly active promoter in the gene construct system so that it is expressed independently from the gene of interest, thus enabling positive selection for successful transfection (David et al. 2005).

Using the constructs and methods according to the invention, MACS can be used for selecting transfected cells characterised by the expression of a marker protein such as an ΔCD4 on their surface, both as a marker for successful transfection and for successful expression of the transfected construct. The present invention relies on the specific expression of a nucleic acid sequence encoding a marker under control of a strong promoter restricted to any subpopulation of cells that is defined by activating a specific promoter, resulting in a specific labelling of this subpopulation with the gene product of nucleic acid sequence encoding the marker, preferably a surface-bound molecule like ΔCD4. Using the constructs and methods according to the invention, it is thus possible to separate virtually any subpopulation of cells that is characterised by activation of a certain promoter from the rest of the cells not using of this specific promoter via MACS.

The specific promoter may for instance simply be introduced into gene construct system such as shown in Fig. 2a, replacing hCN4, and the starting population of cells (e.g. embryonic stem cells, such as murine embryonic stem cells (MESCs) may be transfected with a selection vector containing a coding sequence for resistance to antibiotics and comprising this construct. Resulting clones which have the construct stably integrated into their genomic DNA can then be selected. Those MESCs still have all the characteristics of stem cells and can be expanded in vitro in an unlimited way. When brought to differentiation, only those cells making use of the specific promoter will express the marker protein and simultaneously delete large parts of the construct which they were transfected with and so this subpopulation can be selected.

Preferred lineage-specific or tissue-specific promoters are mesodermal and cardiomyocyte-specific promoters such as Brachyury, Mesp1, Nkx2.5, Gata4, Tbx3, Tbx5, hCN4, cardiac myosin heavy chain (MHC), cardiac myosin light chain (MLC), isl1, and cardiac actin. The isl1 promoter is particularly preferred (Laugwitz et al, 2005).

As a strong promoter controlling the expression of the marker gene, broadly active promoters such as the phosphoglycerate kinase (PGK) promoter are advantageous in the constructs according to the invention. The use of a strong promoter ensures a high expression of the marker gene, while the recombinase gene may also be expressed by a weak specific promoter. Tissue and lineage-specific marking of cells is thus rendered independently of the strength of the specific promoter. Phosphoglycerate kinase (PGK) promoter is particularly preferred as a strong promoter.

In one embodiment, the gene construct system according to the invention comprises a stop cassette containing at least a part of the SV40 polyadenylation signal region. More specific, the stop cassette contains a trimerized SV40 polyadenylation signal or at least parts of the polyadenylation signal or the C-terminal sequence of the yeast His3 gene linked to the SV40 polyadenylation signal region and a synthetic oligonucleotide, wherein ATG is a false translation initiation signal and GTAAGT is a 5' splice donor site (Maxwell, 1989; Lakso, 1992).

In a further aspect, the present invention relates to a vector system comprising the gene construct system according to the present invention. If the elements of the particular gene construct system are included in two or more different constructs, the vector system accordingly comprises two or more vectors.

The term "vector" refers to any nucleic acid molecule suitable to transfer the gene construct system or particular elements of the gene construct system according to the invention into a prokaryotic or eukaryotic cell and thus transfect the cell, either permanently (if the vector or at least of parts of the vector are introduced into the genomic DNA of the cell) or transiently (if the vector remains in an episomal state).

Preferably, the vectors according to the invention additionally comprise a marker gene enabling selection of transfected cells. If two or more vectors comprising two or more constructs comprising the elements of the particular gene construct system according to the invention are used, the two or more vectors preferably contain two or more different marker genes for selection of transfected cells.

Moreover, the invention relates to a host cell or tissue transformed with the vector system according to the invention. A cell or tissue wherein the stop cassette and/or the recombinase gene expression cassette (a) has been deleted due to the recombinase activity is particularly preferred.

In yet another aspect, the present invention provides a method for isolating a pure population of cells of a single cell type comprising the following steps
a) transfecting a starting population of cells with the vector system according to the invention;
b) detecting the marker protein; and
c) separating the cells expressing the marker from the cells which do not express the marker.

In a preferred embodiment, the method comprises the following step:
a) transfecting a starting population of cells with the vector system according to the invention;
b) growing the transfected cells, isolating single clones from the transfected cells and expanding these clones;
c) creating an environment enabling the expression of the nucleic acid sequence encoding the detectable marker comprised in the vector;
d) detecting the marker protein; and
e) separating the cells expressing the marker from the cells which do not express the marker.

If two or more vectors comprising two or more constructs comprising the elements of the particular gene construct system according to the invention are used, the two or more vectors can be transferred into the cells simultaneously or successively.

The starting population of cells may be transfected with the vector system according to the present invention e.g. via physical methods, like microinjection (either into the cytoplasm or the nucleus), particle bombardment ("gene gun"), electroporation, sonoporation, laser irradiation or magnetofection, via strategies based on the endocytosis of the synthetic vector like liposection or polysection or via strategies relying on viral transduction.

In a preferred embodiment, the starting population of cells are stem cells, but generally all cells that can be expanded in vitro and in which subpopulations can be defined by the activation of specific promoters are suitable starting populations within the teaching of the present invention. Embryonic stem cells are particularly preferred.

Embryonic stem cells are cells obtained from the inner mass cells of a blastocyst. They are pluripotent and cannot grow into a whole organism, but they are able to differentiate into cells derived from any of the three germ layers. Adult stem cells are undifferentiated cells found among differentiated cells of a specific tissue and are mostly multipotent cells. Multipotent cells can only turn into some cell type. The term "differentiation" includes any permanent or transient change of the expression profile of a viable cell in response to exogenous or endogenous signals.

As mentioned before, the vector system according to the invention used for transfection may comprise as part of the gene construct system a nucleic acid sequence coding for a protein conferring resistance to an antibiotic as the detectable marker. The clones may then be selected by adding antibiotics to the cultivation medium. Alternatively, a nucleic acid sequence encoding a surface protein marker may be included in the gene construct system comprised in the vector used for transfection, enabling MACS sorting of positively transfected cells. Further, a nucleic acid sequence encoding a fluorescence protein may be included in the gene construct system comprised in the vector used for transfection, enabling FACS sorting or manual picking with the help of a fluorescent microscope of positively transfected cells. Also, the marked cells could be identified by a change in expression profile due to the creation of an environment leading to in vitro differentiation of the cells.

The present invention further relates to a pure population of cells of a single cell type which can be isolated using the methods according to the invention and to pharmaceutical compositions comprising same.

The present invention also relates pharmaceutical compositions comprising the vector system or the host cell or tissue according to the invention.

In another aspect, the present invention relates to the use of the cells isolated by the method according to the invention for tissue engineering, for cell therapy, and for transplantation into an individual. As an individual, mammals are preferred. Human patients are particularly preferred.

The term "tissue engineering" refers to the application of knowledge and techniques from biochemistry, cell biology, physiology, materials science and other sciences on questions regarding the generation and in vitro cultivation of tissue (mostly human tissue) with the aim of enhancing, repairing or replacing damaged tissue of patients. The term "cell therapy" includes any therapeutic strategy that is based on the application of cells of any origin in or on the body of a mammal or the application of derivatives of such isolated cells.

To make sure that no important genomic region is disturbed by a random insertion of the gene construct system when transfecting cells, targeted insertion e.g. via knock-in and homologous recombination, can be used. For that purpose, the gene construct system is flanked by the regions of the genome where the construct is supposed to be integrated, wherein one of those regions is next to a negative selection marker. After positive and negative selection only those cells where a knock-in has occurred will survive and the respective clones can be selected.

Cells derived from an embryonic stem cell clone showing random insertion of the construct or from a knock-in-clone are stem cells and can thus be indefinitely expanded in vitro. They can also be reinserted into a blastocyst of the mammalian species of which the stem cells where generated (in mice most suitable from the murine line of which the used stem cells were produced), this blastocyst can subsequently be transferred into a pregnant mammal and - after several generations - a mammalian line may be obtained that contains the construct in every cell of the body. After isolation of a specific tissue and the production of a single-cell suspension those cells could be isolated that activate a specific promoter. By this means it would be possible to generate virtually any sort of cell.

In case the expression of the marker gene inhibits a normal development of the organ of the mammalian or a normal development of the desired cell lineage in vitro, a version of Cre could be used whose activity additionally depends on the presence of a non-endogenous substance. Versions of Cre whose activity depends on such a non-endogenous substance like the synthetic estrogen antagonist 4-OH-tamoxifen - by adding the sequence of a estrogen ligand binding receptor domain (LBD) to the gene of Flp (Logie and Stewart, 1995) or Cre (Metzger et al., 1995) - have been described. Recently, an enhanced version of an inducible Cre system posttranslationally controlled has been reported by Wunderlich et al. (2001)

Another approach to temporally control the initiation of marker expression is to establish a system with two different recombinases and two pairs of recombinase recognition sites (see Fig.5). The inner recombinase is under the control of the specific promoter and upon expression removes itself, the specific promoter and a stop-cassette from the gene construct system and, simultaneously, brings the gene of the other recombinase under the control of an inducible promoter. If the inducer is added to the system, the second recombinase is expressed and removes itself, the inducible promoter and a second stop-cassette from the gene construct system, and brings the marker under the control of the broadly active Promoter PGK resulting in expression of the marker only in those cells that activated the specific promoter. Recently, a very elaborated system of a Cre-recombinase whose activity depends on the presence of a non-endogenous substance (CreER^{T}) and an inducible promoter (Ah) has been described (Kemp, 2004). The use of said system in the gene construct systems according to the invention would result in a highly specific and temporally controlled expression of the marker and is therefore also contemplated.

If a permanent overexpression of the marker gene is to be avoided, the gene construct system can be placed between two Flp target sites and Flp can also be included between those target sites, either under the control of an inducible promoter (so that after adding the inducer Flp is transcribed) or under the control of a very strong promoter in combination with a version of Flp whose activity depends on the presence of a non-endogenous substance (so that Flp is permanently transcribed but only active in the presence of an inducer, like previously discussed and cited). In both cases the addition of the inducer results in a complete excision of the whole genetic cassette, leaving only one Flp target site behind. In one embodiment of the invention, the inducible promoter is combined with an inducible version of a recombinase (such as (CreER^{T}).

Flp activity could also be transferred to the nuclei of the cells by a version of Flp that is introduced in the medium for cultivation and enters the cells directly For Cre, versions which enter the cells directly have recently been described (Qing Lin, 2004). In this case, no Flp gene would have to be included in the gene construct system, affording only to place the previously described gene construct system between two Flp-target sited without including the gene of Flp itself. Flp activity could also be transferred to the nuclei of the cells by transient transfection with a vector containing only Flp under the control of a strong promoter, or by transferring Flp or Cre mRNA into the cells via electroporation (Van den Plas, 2003). By this means, the overexpression of the marker gene could be stopped after selection of the desired subpopulation by excising the entire gene construct system, leaving behind only one Flp target site at the place of the knock in. It is within the ordinary ability of a person skilled in the art to determine genetic environments (i.e. places where the gene construct system could be integrated) where the method provided by the present invention will work very well and the Flp target site that is left behind will be no obstacle in the development of any cell lineage.

An absolute specific and temporally controlled expression of the marker gene wherein the initiation of marker-expression is induced and, after selection, the marker is eliminated, is contemplated within the scope of the present invention. Said specific and temporally controlled expression of the marker may be achieved by a combination of different recombinases and recombinase target sites with a recombinase that enters the cell directly.

The following Figures and examples shall illustrate the present invention in more detail. The constructs shown in the Figures and the examples given shall not be taken to limit the scope of the invention in any way.

### BRIEF DESCRIPTION OF THE FIGURES

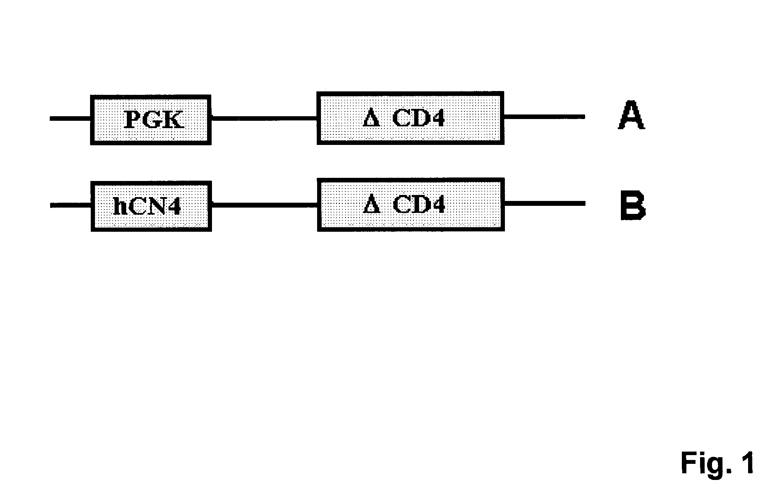

Fig.1-a: Schematic diagram showing a gene construct system comprising a nucleic acid sequence encoding a surface marker (ΔCD4) under the control of a strong, broadly active promoter (PGK).
Fig.1-b: Schematic diagram showing a gene construct system comprising a nucleic acid sequence encoding a surface marker (ΔCD4) under the control of a tissue specific promoter (hCN4).
Fig. 2-a: Schematic diagram showing a gene construct system for positively marking of a subpopulation of cells wherein the gene construct system is comprising a nucleic acid sequence coding for a site-specific-recombinase (Cre) under the control of a specific promoter (hCN4), a stop-cassette (STOP), two recombinase-target-sites (IoxP) symbolized by triangles, a strong promoter (PGK) and the nucleic acid sequence coding for a surface marker (ΔCD4).
Fig. 2-b: Schematic diagram showing the gene construct system of Fig. 2-a after recombinase activity: only one recombinase target-site (IoxP) is left between the strong promoter (PGK) and the nucleic acid sequence coding for the marker (ΔCD4).
Fig. 3: Schematic diagram showing a gene construct system for the negative marking of a subpopulation comprising the nucleic acid sequence coding for a recombinase (Cre) under the control of a specific promoter (hCN4); a strong promoter (PGK), two recombinase target sites (IoxP) symbolized by triangles, a STOP-cassette and a nucleic acid sequence coding for a surface marker (ΔCD4).
Fig. 4: Schematic diagram showing a gene construct system for the negative marking of a subpopulation based on post-transcriptional gene silencing comprising of a specific promoter (hCN4), the nucleic acid sequence coding for a recombinase (Cre), a STOP-cassette, a pair of recombinase target sites (IoxP) symbolized by triangles, two broadly active promoters (PGK), and a nucleic acid sequence coding for a surface-marker (ΔCD4).
Fig. 5: Schematic diagram showing a gene construct system for the temporally inducible positive selection of a subpopulation of cells, comprising a nucleic acid sequence coding for a recombinase (Flp) under the control of a specific promoter (hCN4), a pair of recombinase target sites (FRT) recognized by the lineage-specifically expressed recombinase (symbolized by open triangles), an inducible promoter (Ah), a nucleic acid sequence coding for a recombinase whose activity is dependent on the presence of a non-endogenous substance (CreERT), a pair of recombinase target sites recognized by the inducibly expressed recombinase (loxP, symbolized by filled triangles), three stop-cassettes, a strong broadly active promoter (PGK), and a nucleic acid sequence coding for a surface marker (ΔCD4).
Fig. 6: Schematic diagram showing a gene construct system for the inducible shutdown of the expression of the nucleic acid sequence coding for the marker after selection of the subpopulation, wherein the gene construct system comprises a specific promoter (hCN4), a nucleic acid sequence coding for a recombinase (Flp), a pair of recombinase recognition sites recognized by the tissue-specifically expressed recombinase (FRT) symbolized by open triangles, a STOP-cassette, a strong promoter (PGK), an inducible promoter (Ah), a nucleic acid sequence coding for a recombinase whose activity is dependent on the presence of a non-endogenous substance (CreER^{T}), two recombinase target sites recognized by the inducebly expressed recombinase (IoxP) symbolized by filled triangles, and a nucleic acid sequence coding for a surface marker (ΔCD4).

It is noted that the triangles (open and filled) are intended only to reflect the position of the recombinase recognition/target sites. They do not indicate the orientation of the recombinase recognition/target site sequences. That is, both orientations (3' to 5' and 5' to 3') may be possible.

### EXAMPLES

### Example 1: Expression of ΔCD4 under control of a strong endogenous promoter

MACS is capable of enriching even very small subpopulation of cells as long as they express ΔCD4 in a reasonable amount on the cell surface. To confirm that mammalian endogenous promoters can provide an expression level sufficient for MACS-selection, David et al. (2005) transfected murine embryonic stem cells (MESCs) with construct A (Fig. 1a). The ΔCD4 fragment had been derived via proofreading PCR from pMACS 4.I (Miltenyi Biotech GmbH). This construct leads to an expression of ΔCD4 depending on the very strong and endogenous PGK-promoter. It was ligated into in a selection vector with a gene coding for resistance against geneticin and several clones transfected with the resulting vector were selected.

FACS analysis using a FITC-labeled anti-CD4 antibody (Miltenyi Biotech GmbH) revealed that 37.7 % up to 53.8 % of the cells were ΔCD4 positive. In order to reduce the percentage of ΔCD4 positive cells to a more realistic number with respect to applications using specific promoters leading to only a small percentage of the cells expressing ΔCD4, the labelled cells were diluted to 5.9 % with native differentiated MESCs before MACS purification. This initial population of differentiated cells displaying the ΔCD4 antigen by FACS was enriched to 96.2 % validated by FACS. Likewise, when undifferentiated ΔCD4 positive cells were used at initial percentages between 3.8% and 15.8%, purities between 97.6 % and 98.4 % of cells were achieved. So it is clear that MACS can enrich a subpopulation of cells with a very small initial percentage of about 3 % up to 97-98% (David et al., 2005)

### Example 2: Expression of ΔCD4 under control of a cell type-specific promoter

In order to select a specific cell lineage, David et al. (2005) constructed several vectors which lead to an expression of ΔCD4 dependent on the activation of tissue specific promoters, like hCN4 ( Fig. 1b), Mesp1 or Nkx2.5. MESCs were transfected with the respective constructs comprised in a selection vector. It turned out that most of the tested endogenous tissue-specific promoters do not lead to an expression of ΔCD4 sufficient for MACS purification. However, the possibility cannot be ruled out that there are several endogenous promoters, especially in non-cardiac cell-lineages, that are strong enough for this purpose.

### Example 3: Tissue-specific expression of ΔCD4 mediated by Cre activity

According to the present invention, in order to render the amount of ΔCD4 expression independent of the strength of the endogenous promoter, the ΔCD4-based MACS-purification system described above may be combined with the very well established site-specific-recombinase systems, in this case the Cre/IoxP-system.

For that purpose, the construct shown in Fig. 2a was constructed which mediates an expression of Cre by the weak, tissue-specific promoter hCN4. This construct was ligated into a selection vector, MESCs were transfected with said vector, and several clones were selected. Cells that activate the endogenous hCN4-promoter will consequently also express Cre and thus have their DNA recombined at the two IoxP-sites resulting in the construct shown in Fig. 2b. Upon recombination of the genomic DNA, the Cre gene is excised and, simultaneously, ΔCD4 is expressed under the control of the strong promoter PGK (which in construct C is silenced by a STOP-cassette) in high amounts.

For providing the construct shown in Fig. 2b, construct A shown in Fig. 1 was restricted using Age1, and the loxP sequences were introduced with the help of mutagenesis PCR. The cassette containing the two Stop-sequences, the hCN4-promoter and the Cre gene was constructed separately and ligated into the Age1 site. The inverse-stop-cassette guarantees that the PGK-promoter is silenced properly, independent of the orientation of the central construct.

### Example 4: Non-specific expression of ΔCD4 mediated by Cre activity

Another strategy to overcome the disadvantage of a permanent overexpression of ΔCD4 is to use the gene construct system shown in Fig. 3.

MESCs are transfected with this construct comprised in a selection vector and resulting clones, preferentially knock-in-clones are selected. Transfection with a vector comprising this construct results in an overexpression of ΔCD4 in all cells of the cell population which do not make use of the tissue-specific promoter (in this example hCN4). When during differentiation a subpopulation of cells starts to make use of the promoter, Cre is expressed and PGK is excised and replaced by a STOP-cassette so that ΔCD4 expression comes to an end. MACS purification of ΔCD4 expressing cells results in purifying all cells that do not activate the specific promoter leaving behind those that activate the specific promoter.

### CITATIONS

Belteki, G., Gertsenstein, M., Ow, D.W., and Nagy, A. (2003). Site- specific cassette exchange and germline transmission with mouse. ES cells expressing phiC31 integrase. Nat. Biotechnol. 21, 321-324.
David, Gröbner, Franz (2005). MACS Purification of Differentiated ES Cells Stably Expressing Truncated Human CD4 as Surface Marker. Stem cells 23 (4)
Groth, A.C., Olivares, E.C., Thyagarajan, B., and Calos, M.P. (2000). A phage integrase directs efficient site-specific integration in human cells. Proc. Natl. Acad. Sci. USA 97, 5995-6000.
Hannon. (2002). RNA interference. Nature 418 244-251
Kemp R, Ireland H, Clayton E, Houghton C, Howard L, Winton DJ. Nucleic Acids Res. 2004 Jul 1;32(11):e92. Elimination of background recombination: somatic induction of Cre by combined transcriptional regulation and hormone binding affinity.
Kunath T, Gish G, Lickert H, Jones N, Pawson T, Rossant J. (2003). Transgenic RNA interference in ES cell-derived embryos recapitulates a genetic null phenotype. Nat Biotechnol. 2003 May;21(5):559-61.
Lakso M, Sauer B, Mosinger B Jr, Lee EJ, Manning RW, Yu SH, Mulder KL, Westphal H. Targeted oncogene activation by site-specific recombination in transgenic mice. Proc Natl Acad Sci U S A. 1992 Jul 15;89(14):6232-6.
Laugwitz, Moretti, Lam, Gruber, Chen, Woodard, Li, Cai, Minlu, Reth, Platoshyn, Yuan, Evans, Chien. (2005) Postnatal isl1+ cardioblasts enter fully differentiated cardiomyocyte lineages. Nature 433, 647 - 653;
Logie, C., and Stewart, F. (1995). Ligand-regulated site-specific recombination. Proc. Natl. Acad. Sci. USA 92, 5940-5944.
Maxwell IH, Harrison GS, Wood WM, Maxwell F. A DNA cassette containing a trimerized SV40 polyadenylation signal which efficiently blocks spurious plasmid-initiated transcription. Biotechniques. 1989 Mar;7(3):276-80
Metzger, D., Clifford, J., Chiba, H., and Chambon, P. (1995). Conditional site-specific recombination in mammalian cells using a ligand-dependent chimeric Cre recombinase. Proc. Natl. Acad. Sci. USA 92, 6991-6995.
Müller M, Fleischmann BK, Selbert S, Ji GJ, Endl E, Middeler G, Muller OJ, Schlenke P, Frese S, Wobus AM, Hescheler J, Katus HA, Franz WM. Selection of ventricular-like cardiomyocytes from ES cells in vitro. FASEB J. 2000 Dec;14(15):2540-8.
Qing Lin, Daewoong Jo, Kassatihun D Grebre-Amlak, and H Earl Ruley (2004). Enhanced cell-permeant Cre protein for site-specific recombination in cultured cells. BMC Biotechnol. 2004; 4: 25.
Sauer B, McDermott J. DNA recombination with a heterospecific Cre homolog identified from comparison of the pac-c1 regions of P1-related phages. Nucleic Acids Res. 2004 Nov 18;32(20):6086-95.
Schindehütte J, Fukumitsu H, Collombat P, Griesel G, Brink C, Baier PC, Capecchi MR, Mansouri A. In vivo and in vitro tissue-specific expression of green fluorescent protein using the cre-lox system in mouse embryonic stem cells. Stem Cells. 2005;23(1):10-5.
Van den Plas D, Ponsaerts P, Van Tendeloo V, Van Bockstaele DR, Bememan ZN, Mer-regaert J. (2003). Efficient removal of LoxP-flanked genes by electroporation of Cre-recombinase mRNA. Biochem Biophys Res Commun. 2003 May 23;305(1):10-5.
Wunderlich, F.T., Wildner, H., Rajewsky, K., and Edenhofer, F. (2001). New variants of inducible Cre recombinase: a novel mutant of Cre-PR fusion protein exhibits enhanced sensitivity and an expanded range of inducibility. Nucleic Acids Res. 29, E47.

## Claims

1. Gene construct system comprising the following elements
a) a recombinase gene expression cassette comprising
i) a nucleic acid sequence encoding a recombinase; and
ii) a lineage-specific or tissue-specific promoter operably linked to said nucleic acid sequence encoding the recombinase;
b) a strong promoter;
c) a nucleic acid sequence encoding a detectable marker;
d) at least one stop cassette; and
e) at least two recombinase target sites;
wherein the at least one stop cassette is arranged between the strong promoter and the nucleic acid sequence encoding the detectable marker.

2. The gene construct system according to claim 1, wherein the at least one stop cassette is located between the at least two recombinase target sites.

3. The gene construct system according to claim 2, wherein on activation of the lineage-specific or tissue-specific promoter, the recombinase is expressed and removes the stop cassette located between the recombinase target sites, resulting in the expression of the nucleic acid sequence encoding the detectable marker under control of the strong promoter.

4. The gene construct system according to claim 1, wherein the recombinase gene expression cassette and the at least one stop cassette are located between the at least two recombinase target sites.

5. The gene construct system according to claim 4, wherein on activation of the lineage-specific or tissue-specific promoter, the recombinase is expressed and removes the recombinase gene expression cassette and the at least one stop cassette, resulting in the expression of the nucleic acid sequence encoding the detectable marker under control of the strong promoter.

6. Gene construct system comprising the following elements
a) a recombinase gene expression cassette comprising
(i) a nucleic acid sequence encoding a recombinase; and
(ii) a lineage-specific or tissue-specific promoter operably linked to said gene encoding the recombinase;
b) a strong promoter;
c) a nucleic acid sequence encoding a detectable marker;
d) at least one stop cassette; and
e) at least two recombinase target sites;
wherein one of the at least two recombinase target sites is arranged between the strong promoter and the nucleic acid sequence encoding the detectable marker.

7. The gene construct system according to claim 6, wherein the recombinase gene expression cassette and the strong promoter are located between the at least two recombinase target sites.

8. The gene construct system according to claim 7, wherein on activation of the lineage-specific or tissue-specific promoter, the recombinase is expressed and removes the recombinase gene expression cassette and the strong promoter located between the recombinase target sites, resulting in a stop of expression of the nucleic acid sequence encoding the detectable marker.

9. The gene construct system according to any of claims 1-8, wherein the elements comprised by said system are located on at least two different constructs.

10. The gene construct system according to claim 9, wherein element (a) is comprised in the first of the at least two different constructs and elements (b)-(e) are comprised in the second of the at least two different constructs, and further
wherein the at least one stop cassette is arranged between the strong promoter and the nucleic acid sequence encoding the detectable marker and said stop cassette is flanked by the at least two recombinase target sites.

11. The gene construct system according to claim 10, wherein the nucleic acid sequence encoding the recombinase comprised in element (a) is flanked by another two recombinase target sites.

12. The gene construct system according to any of claims 1-11, wherein the recombinase is a Cre recombinase and the recombinase target sites are loxP sites.

13. The gene construct system according to any of claims 1-11, wherein the recombinase is a Flp recombinase and the recombinase target sites are FRT sites.

14. The gene construct system according to any of claims 1-13, wherein the marker is a surface-bound protein.

15. The gene construct system according to claim 14, wherein the surface-bound protein is an intracellularly truncated CD4 (ΔCD4).

16. The gene construct system according to any of claims 1-15, wherein the lineage-specific or tissue-specific promoter is a cardiomyocyte-specific promoter.

17. The gene construct system according to any of claims 1-16, wherein the strong promoter is a phosphoglycerate kinase (PGK) promoter.

18. The gene construct system according to any of claims 1-17, wherein the at least one stop cassette is containing at least a part of the SV40 polyadenylation signal region.

19. Vector system comprising the gene construct system of any of claims 1-15.

20. Host cell or tissue transformed with the vector system of claim 19.

21. The cell or tissue according to claim 20, wherein the stop cassette and/or the recombinase gene expression cassette (a) has been deleted due activity of the recombinase.

22. Method for isolating a pure population of cells of a single cell type comprising the following steps
a) transfecting a starting population of cells with the vector system of claim 19;
b) detecting the marker protein; and
c) separating the cells expressing the marker from the cells which do not express the marker.

23. The method according to claim 22, wherein the starting population of cells are stem cells.

24. The method according to claim 23, wherein the stem cells are embryonic stem cells.

25. The method according to any of claims 22-24, wherein the separation in step (e) is done by magnetic cell sorting (MACS).

26. Pure population of cells of a single cell type isolated by the method according to any of claims 22-25.

27. Pharmaceutical composition comprising an adequate pharmaceutical carrier and the gene construct system of any of claims 1-18, and/or the vector system of claim 19, and/or the host cell of claim 20 or 21, and/or the pure population of cells of claim 26.

28. Use of the gene construct system of any of claims 1-18, and/or the vector system of claim 19, and/or the host cell or tissue of claim 20 or 21, and/or the pure population of cells of claim 26 for tissue engineering.

29. Use of the gene construct system of any of claims 1-18, and/or the vector system of claim 19, and/or the host cell of claim 20 or 21, and/or the pure population of cells of claim 26 for cell therapy.

30. Use of the gene construct system of any of claims 1-18, and/or the vector system of claim 19, and/or the host cell or tissue of claim 20 or 21, and/or the pure population of cells of claim 26 for transplantation into an individual.
